# EUROPEAN PATENT APPLICATION

(11) **EP 2 314 154 A1**
(43) Date of publication of application: **27.04.2011**
(21) Application number: 09174031.6
(22) Date of filing: 26.10.2009
(51) Int. Cl.: A01K 11/00, A61K 9/00

(54) **Bolus System**

(71) Applicant: Nederlandse Organisatie voor Toegepast -Natuurwetenschappelijk Onderzoek TNO, 2628 VK Delft (NL)
(72) Inventor: Sewalt, Karl Everhardus, 3119 BT Schiedam (NL); van Tilburg, Mark Wilhelmus Theresia, 5691 ZC Son (NL)
(74) Representative: Hatzmann, Martin

(57) **Abstract**

Method for installing a bolus system into a stomach or other organ of an animal, comprising the steps of (a) providing at least two boluses (1, 5) comprising magnetic attraction means (4, 6) for mutual attracting each other when present in each other's proximity, (b) administering at least one of the boluses (1) to the animal at a first moment and (c) administering always at least one of the boluses (5) to the animal at later moments. At least one of the boluses administered to the animal at the first moment may include data exchange means (3) and possibly data collection means related to the health of the animal.

## Description

The identity and physiological condition of livestock is of paramount importance to owners, buyers, and processors of livestock. It is known to visually identify livestock using brands or ear tags. It is also known to identify livestock using radio frequency identification (RFID) tags, e.g., in the form of an ear tag, an injectable tag, or an ingestible bolus which is intended to be received in a stomach, e.g. the reticulum (second pre-stomach) of a ruminant. By including a sensor with the RFID tag, it is possible to collect indications of physiological characteristics, e.g., temperature etc. Besides for animal identification, monitoring etc., boluses may be used for internal delivery of nutrients, vitamins, medicines etc.

Known boluses, which may include RFID chips etc. for animal identification and/or monitoring, are too large to administer to newborn ruminant calves (0 - 3 days). The bolus may be stuck in and/or harm the oesophagus; newborn calves, besides, have a limited peristalsis. Known are "miniboluses" for e.g. lams and goats. Such miniboluses, however, are too small to be used for cows, as they, due to their small volume and/or low density, may be lost during ruminating, end up in another stomach or be lost via the excrements.

One aim of the present invention is to provide a bolus system which is adaptable in size and/or density (weight), in order to be suitable to be used for newborn bovine animals (e.g. calves) as well as for mature bovine animals.

Another aim of the invention is to provide a bolus system comprising single boluses having limited dimensions, which can administered individually at subsequent points of time, thus enabling enhanced and more secure administration and installation in the (small) animal's reticulum, and enabling that, by delayed administration of additional boluses and "magnetic coagulation" within the stomach of the (growing) animal the bolus system, having then more interconnected boluses, has sufficient volume and density to prevent that the bolus system unintentionally disappears from the reticulum.

According to the invention, a bolus system, intended for installing into a stomach or other organ of an animal, is provided, which system preferably comprises at least one bolus having magnetic attraction means for attracting one or more other boluses when present in each other's proximity. The magnetic attraction means may comprise a permanent magnet in at least one bolus. At least one (other) bolus may comprise a magnetic (e.g. a ferromagnetic) attraction member or may be formed by another permanent magnet. Small neodymium magnets may be used, which may be coated by a plastic or ceramic outside layer which is inert for the biochemical compounds and processes within the animal's stomach.

Known as such, from US2004/0155782 and US2006/0132317, is that at least one bolus may include data exchange means and possibly data collection means, sensors, detectors etc.

A method for installing a bolus system into a stomach or other organ of an animal, preferably comprises the steps of:
- providing at least two boluses comprising magnetic attraction means for mutual attracting each other when present in each other's proximity;
- administering at least one of said boluses to said animal at a first moment;
- administering always at least one of said boluses to said animal at later moments.

Preferably at least one of the boluses administered to the animal at the first administration moment (e.g. administered to a newborn calf) includes data exchange means, e.g. an RFID tag for animal identification, and, possibly, data collection means, e.g. for animal health monitoring. Moreover, such bolus may be used for internal delivery of nutrients, vitamins, medicines etc.

Hereinafter, the invention will be elucidated further with reference to some figures, wherein:
- Figure 1: shows, in cross sectional view, an exemplary embodiment of the bolus system according to the invention, including a central bolus and two satellite boluses;
- Figure 2: shows, in perspective view, a central bolus to be administered solely to a newborn animal;
- Figure 3: shows the central bolus shown in figure 2, provided with one satellite bolus, interconnected by mutual magnetic attraction force;
- Figure 4: shows the bolus system shown in figure 3, provided with a second satellite bolus, administered to the animal when growing up.

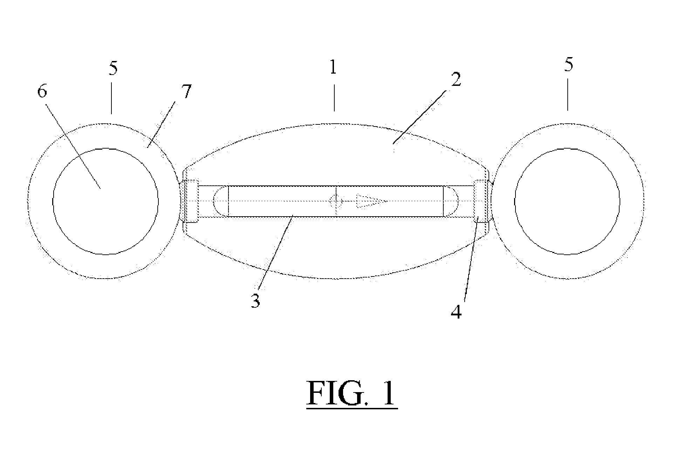

Figure 1 shows a central bolus 1, having a body 2 made from e.g. a ceramic material, which covers an RFID chip 3 or another data transmission chip, possibly including capabilities for data collection related to the animal's health etc., as e.g. suggested in US2004/0155782 and US2006/0132317. The bolus, moreover, includes two magnets 4. This central bolus 1, may be administered to a newborn calf, within 1 - 3 days after its birth, in order to be remotely identifiable.

After a certain time the calf will have become larger. To avoid that the central bolus might be disappear from the reticulum of the calf, one satellite bolus 5 will be administered to the calf. Such a satellite bolus 5 includes a body 6 which is covered by e.g. a ceramic layer 7. The "magnetic attraction" body 6 is made of a permanent magnetic material like neodymium, or is made of a ferromagnetic material like iron, nickel or ferrite. After the satellite bolus 5 has been arrived in the reticulum of the calf, the satellite bolus 5 will be connected, by their mutually attracting magnetic forces, to the central bolus 1 which was administered to the calf within 3 days after its birth. By this magnetic connection of the boluses 1 and 5 the total volume and density of the total bolus system 1-5 may be sufficient to prevent ejection of the bolus system from the animal's reticulum.

Still some time later, when the calf has become a young cow, a second satellite bolus 5 may be administered to the animal, which, again, is attracted by the central bolus 1, after which the volume and density of the bolus system 5-1-5, has been increased such that the whole can not leave the reticulum.

It is also possible to feed individually all separate parts, i.e. the central bolus 1 and the satellite boluses 5, within 1 - 3 days after the calf is born, instead of waiting until the calf has grown.

It is noted further that more (than two) satellite boluses may be fed to the animal. More satellite boluses 5 may be attracted one another by the magnetic field or fields generated in the central bolus 1 and possible in one or more satellite boluses.

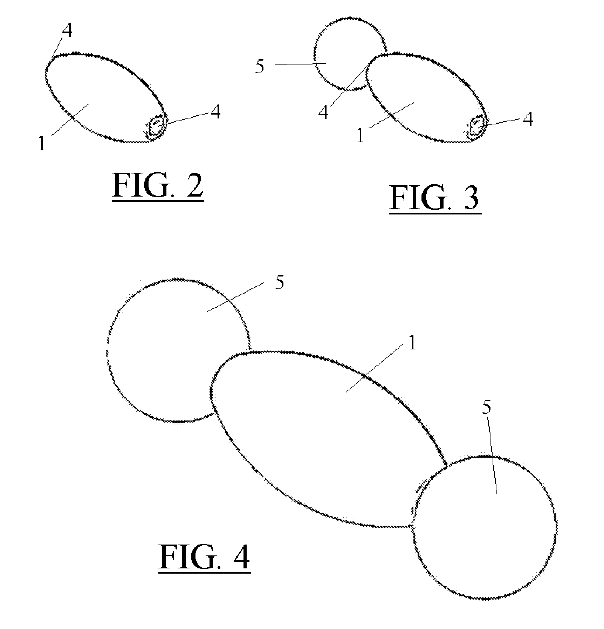

Figure 2 shows again the central bolus 1, to be administered to a newborn animal, including magnets 4 extending to its outer surface (however preferably covered by a suitable coating of cover).

Figure 3 shows the central satellite bolus 1, shown in figure 1, administered to the animal when newly born, and one satellite bolus 5 coagulated, after being administering after some months to the animal, and coagulating with the central bolus 1 by their mutual magnetic attraction force of one of the permanent magnets 4.

Figure 4 shows the bolus system, completed with a second satellite bolus 5, administered to the animal when it has become more or less mature, and thus forming a bolus system which has sufficient volume and density to prevent that it can be lost from the reticulum.

It is noted that, in particular when the satellite boluses include magnetic bodies having permanent magnetic characteristics, e.g. when they are made of neodymium, it is possible, when desired to add more (than two) satellite boluses to the bolus(es) already in the reticulum.

It is noted that in US2004/0155782 and US2006/0132317 applying a permanent magnet, included in a bolus, is known as such. However, the aim of that permanent magnet, mounted in the bolus at a location separated from the transponder electronics, is to reduce the risk of hardware disease in the bolus host by attracting metal objects.

## Claims

1. Bolus system, intended for installing into a stomach or other organ of an animal, comprising at least one bolus (1, 5) having magnetic attraction means (4, 6) for attracting one or more other boluses (5) when present in each other's proximity.

2. Bolus system according to claim 1, comprising at least two boluses (1, 5) having magnetic attraction means (4, 6) for attracting one another when present in each other's proximity.

3. Bolus system according to claim 1 or 2, at least one bolus (1) comprising at least one permanent magnet (4).

4. Bolus system according to claim 3, at least one bolus (5) comprising a magnetic attraction member (6).

5. Bolus system according to any preceding claim, at least one of said boluses (1, 5) including data exchange means (3).

6. Bolus system according to any preceding claim, at least one of said boluses (1, 5) including data collection means.

7. Bolus system according to any preceding claim, at least one of said boluses (1, 5) being arranged for internal delivery of nutrients, vitamins, medicines etc.

8. Method for installing a bolus system into a stomach or other organ of an animal, comprising the steps of:
- providing at least two boluses (1, 5) comprising magnetic attraction means (4, 6) for mutual attracting each other when present in each other's proximity;
- administering at least one of said boluses (1) to said animal at a first moment;
- administering always at least one of said boluses (5) to said animal at later moments.

9. Method according to claim 8, at least one of the boluses administered to the animal at said first moment includes data exchange means (3).

10. Method according to claim 9, at least one of the boluses administered to the animal at said first moment includes data collection means.

11. Method according to claim 8, at least one of said boluses being arranged for internal delivery of nutrients, vitamins, medicines etc.
